# EUROPEAN PATENT SPECIFICATION

(11) **EP 3 984 469 B1**
(45) Date of publication and mention of the grant of the patent: **21.05.2025**
(21) Application number: 20822627.4
(22) Date of filing: 12.06.2020
(51) Int. Cl.: A61B 10/06, A61B 10/04, A61B 18/08

(54) **SAMPLING SYSTEM**
PROBENNAHMESYSTEM
SYSTÈME D'ÉCHANTILLONNAGE

(30) Priority: 14.06.2019 HK 19125288
(43) Date of publication of application: 20.04.2022
(73) Proprietor: Tam, Chi Chun Terence, Hong Kong (CN)
(72) Inventor: YUEN, Mae Ann Michele, Hong Kong (CN); TAM, Chi Chun Terence, Hong Kong (CN)
(74) Representative: HWP Intellectual Property
(86) International application number: PCT/CN2020/095843
(87) International publication number: WO 2020/249090

(56) References cited:
- WO-A1-2019/103694
- CN-A- 101 365 388
- CN-A- 102 258 393
- CN-A- 102 309 348
- CN-A- 102 379 727
- CN-U- 204 072 177
- CN-U- 204 468 156
- JP-A- 2000 189 435
- JP-A- H1 119 086
- US-A- 5 133 360
- US-A- 5 217 458
- US-A- 5 848 978
- US-A1- 2002 169 362
- US-A1- 2005 113 854
- US-B1- 6 443 909

## Description

### TECHNICAL FIELD

Embodiments of the present disclosure relates to the technical field of medical devices, and particularly to a sampling system.

### BACKGROUND

Endoscopy is an approach to obtain the type of lesion in a human body, which includes sampling a tissue in the human body by a sampling system to obtain a tissue sample and then examining the tissue sample to determine the type of lesion in a lesion site in the human body. During operation, a surgeon delivers the sampling system into a lesion site in a human body, acquires a sample from the tissue in the lesion site in the human body by an acquiring device on the sampling system to obtain a tissue sample, and examines the tissue sample, to acquire the cause and type of lesion in the lesion site in the human body.

US 2002/169362 A1 discloses an operating instrument insertion apparatus capable of easily inserting an operating instrument into the tissue. The operating instrument insertion apparatus includes a hollow tubular member for receiving an operating instrument therein, and a plurality of scoop-like members provided at a distal end of the hollow tubular member. The plurality of scoop-like members can be opened in such a manner that distal ends of the scoop-like members are moved away from each other in a direction transverse to an axial direction of said hollow tubular member, and the plurality of scoop-like members can be closed in such a manner that the distal ends of the scoop-like members are moved toward each other in this transverse direction, wherein the scoop-shape members in the opened condition cooperate so that an outside diameter formed thereby is no greater than an outside diameter of the hollow tube member.

JP H11-019086 A discloses that: a fulcrum shaft is bridged to a distal side of a supporting piece of a supporting utensil which is fixed to a distal end of a sheath. On the fulcrum shaft, link pieces of forceps pieces are turnably fitted, and an insulating spacer is fitted in a space between the link pieces. To the link pieces, other link pieces are connected under a pantograph state, and to the rear ends of the link pieces, a distal end of a longitudinal member, which can advance or retract in the longitudinal direction X along a lumen in the sheath, is connected. The forceps pieces are turnably fitted in the fulcrum shaft in such a manner that cup- shaped recesses may be confronted to each other, and electrodes are formed at the tip end side edges of the recesses of the forceps pieces, and a distal end ofa conductive wire is electrically connected.

US 5,217,458 A discloses a bipolar biopsy device for removing tissue samples for biopsy purposes or other purposes. The bipolar biopsy device has an elongated flexible end and a lumen extending therebetween. A cutting head is mounted on the distal end and has a hollow fixed member containing an electrode having an electrical surface thereon and a hollow cup-shaped moveable relative to the fixed member. The electrode surfaces are electrically connected to an outside voltage source. A handle is affixed to the proximal end and a core wire is affixed to the handle which extends through the lumen and is affixed to the movable hollow cup-shaped member. The core wire manipulated by way of the handle facilitates the movement of the movable cup-shaped member. Tissue samples are obtained by positioning the electrode surfaces close to each other about the tissue sample. An arc is created to break tissue down by applying a voltage to the electrode surfaces. The cut tissue remains within the cup-shaped members as the device is withdrawn from the body for later biopsy purposes.

US 5,133,360 A discloses a biopsy instrument which has a retractable cutting wire or filament looped external to the innermost surface of the distal end of the tissue cutting cylinder. Forceful retraction of the cutting wire will cause displacement and closure of a loop of wire over the distal margin of the cored tissue biopsy, thus freeing the biopsy specimen in its entirety from its original locus and allowing removal of the entire cored biopsy from the body substantially without stretching the specimen along its length.

US 5,848,978 A discloses a surgical core biopsy apparatus, having a hollow elongated member with an axis and a leading end, a sharpened edge at a portion of the leading end for cutting tissue along the axis, an actuator, and a cutting edge, linked to the actuator, being movable along a path including a transverse component to the axis, effective for severing tissue along an the path. The path is preferably an arcuate path, the cutting device being pivoted about an axis transverse to the axis of said hollow elongated member at said leading end. The actuator preferably acts by way of a compression force transmitted along the axis by a compression member, from a handle portion to the cutting edge. The elongated member is preferably a tube having two or more lumens, a first large centrally located lumen for accommodating a tissue core sample, and at least one other eccentrically located rectangular cross section lumen containing the compression member. The biopsy apparatus may be used, for example, to obtain a percutaneous excision breast biopsy from a tumor whose location is marked with a radiopaque guide wire.

US 2005/113854 A1 discloses a surgical instrument for removal of a conical section the cervix for pathological examination is disclosed. The device includes a circular knife having a plurality of double-edged blades, the edges of adjacent blades enclosing against one another when the device is operated from unengaged to engaged positions. A hollow plunger is further disclosed used to actuate the circular knife. Novel pivot devices are additionally included to provide translational movement of the hollow plunger bar. A pronged stabilization rod is disclosed to prevent the circular knife from moving away from the target tissue during a cutting stroke. The device is an improvement over prior devices and procedures in that it provides a precision conical tissue sample ideal for analysis with minimal resulting bleeding.

WO 2019/103694 A1 discloses a balloon-anchored biopsy device.

At present, the sampling device on the sampling system is usually forceps or needle aspiration. However, the sample is often too shallow and/or small when the tissue sample is repeatedly taken by forceps or by needle aspiration; and if large-size and deep sampling (such as cryobiopsy) is adopted, significant bleeding is often caused, and the operation is not safe and reliable.

### SUMMARY

The object of the embodiments of the present application is to provide a sampling system with safe and reliable operation.

To solve the above technical problem, the following technical solution is adopted in the present invention. A sampling system is provided, which includes:
a sampling unit, configured for acquiring a tissue sample;
a control unit, configured for controlling the working state of the sampling unit and including a first adjustment assembly, where the first adjustment assembly includes a first adjustment knob and an actuating string, and the actuating string is rigid; and
a connecting tube, assembled to the control unit and having at least one end running through the control unit, and one end of the connecting tube being fixedly assembled to the sampling unit,
wherein one end of the actuating string is fixed to the first adjustment knob and an other end of the actuating string is fixed to the sampling unit; and due to the rigidity of the actuating string the sampling unit is controllable to be opened or closed by controlling the first adjustment knob, wherein
the sampling system further comprises a balloon unit having a balloon and a metal ring, wherein the balloon is mounted around the metal ring, the metal ring is mounted around the connecting tube, and the balloon unit is located between the sampling unit and the control unit.

Furthermore, the sampling unit comprises a sampling sheath, a support portion and a heating member, wherein one end of the sampling sheath inserts into the connecting tube and connected to the connecting tube, and an other end of the sampling sheath is connected to the support portion; the support portion is deformable upon an external force, and the heating member is mounted around the sampling sheath; and
the one end of the actuating string is fixed to the first adjustment knob and the other end of the actuating string is fixed to the heating member; the actuating string is controllable to move by controlling the first adjustment knob, whereby the heating member is controlled to be opened or closed, which in turn drives the support portion to be opened or closed.

Further, the connecting tube comprises a communicating tube, having at least one end extending through the control unit, wherein the one end of the sampling sheath inserts into the communicating tube and is connected to the communicating tube; and the communicating tube is provided with a receiving passage and an aperture, wherein the aperture is located in the control unit.

Optionally, the control unit comprises a conductive component having a cable sheath and a cable, wherein
the cable sheath is fixedly mounted in the control unit, and the cable is partly located in the cable sheath and partly in the receiving passage, and the cable has one end electrically connected to the outside and an other end extending through the aperture and the receiving passage and electrically connected to the heating member.

Additionally, the connecting tube further comprises an endoscope tube and an outer sleeve, wherein
at least one end of the endoscope tube extends through the control unit, and one end of the endoscope tube is assembled to the sampling sheath, the endoscope tube is movable relative to the control unit, and the outer sleeve is located outside the control unit and has one end assembled to the control unit; and
the outer sleeve is mounted around the communicating tube, and the communicating tube is mounted around the endoscope tube.

Optionally, the connecting tube further comprises a gas-liquid tube located between the outer sleeve and the communicating tube, and
both the balloon and the metal ring are mounted around one end of the gas-liquid tube adjacent to the sampling unit.

Optionally, the control unit further comprises a linear rack and a second adjustment assembly,
the linear rack is fixed to the communicating tube and the linear rack is parallel to the communicating tube; and
the second adjustment assembly comprises a second adjustment knob and a third adjustment gear, the second adjustment knob is assembled to the third adjustment gear, and the third adjustment gear is drivable by the second adjustment knob to rotate; and
wherein the second adjustment knob is assembled to the control unit, and the second adjustment knob is partially located in the control unit; and the third adjustment gear is located in the control unit and engaged with the linear rack.

Optionally, the control unit further comprises a conducting assembly having a balloon gas-liquid joint, wherein the balloon gas-liquid joint is communicated with the gas-liquid tube, and the balloon gas-liquid joint is provided with a balloon gas-liquid exchange port thereon;
the gas-liquid tube is provided with a first gas-liquid guide hole;
an inner wall of the balloon is connected to the gas-liquid tube and is provided with a first gas-liquid acquiring hole; and
the first gas-liquid acquiring hole communicates with the first gas-liquid guide hole, and the balloon gas-liquid exchange port communicates with the first gas-liquid guide hole and an external environment.

Optionally, the conducting assembly further comprises a body gas-liquid joint, wherein the body gas-liquid joint is connected to the gas-liquid tube, and the body gas-liquid joint is provided with a body gas-liquid exchange port thereon;
the gas-liquid tube is provided with a second gas-liquid guide hole, and the second gas-liquid
guide hole communicates with an internal environment; and
the second gas-liquid guide hole communicates with the body gas-liquid exchange port, and the body gas-liquid exchange port communicates with the second gas-liquid guide hole and the external environment.

Embodiments of the present application have the following beneficial effects. The control unit and the sampling unit are connected through a connecting tube, the control unit is provided with a first adjustment assembly, and the first adjustment assembly includes a first adjustment knob and an actuating string. Two ends of the actuating string are respectively connected to the first adjustment knob and the sampling unit. Due to the rigidity and high hardness of the actuating string, by controlling the first adjustment knob, the movement of the actuating string is controlled to be pulled or loosened, so that the sampling unit can be opened and closed to enable the cutting and acquisition by sampling unit with safe and reliable operations.

### BRIEF DESCRIPTION OF THE DRAWINGS

FIG. 1 is a schematic structural view of a sampling system in an embodiment of the present application;
FIG. 2 is another schematic structural view of the sampling system shown in FIG. 1;
FIG. 3 is a cross-sectional view of the sampling system shown in FIG. 1;
FIG. 4 is another cross-sectional view of the sampling system shown in FIG. 1;
FIG. 5 is a further cross-sectional view of the sampling system shown in FIG. 1;
FIG. 6 is a partially enlarged view of the sampling system shown in FIG. 2;
FIG. 7 is a reference view showing the state of a sampling unit in the sampling system shown in FIG. 6;
FIG. 8 is another reference view showing the state of the sampling unit in the sampling system shown in FIG. 6;
FIG. 9 is another reference view showing the state of the sampling unit in the sampling system shown in FIG. 7; and

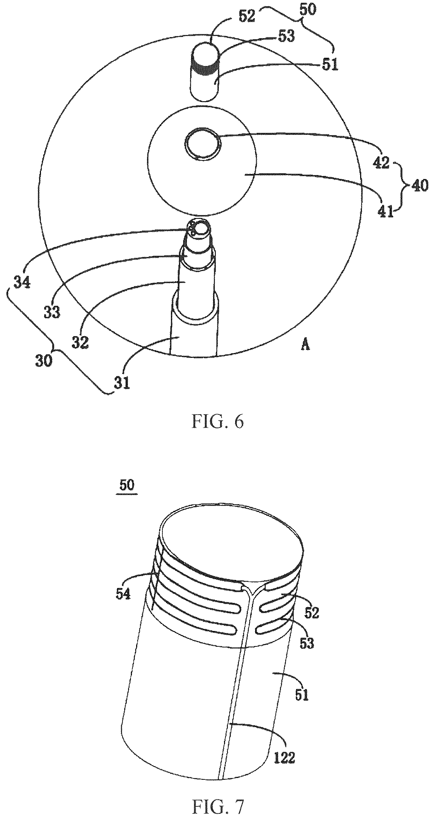

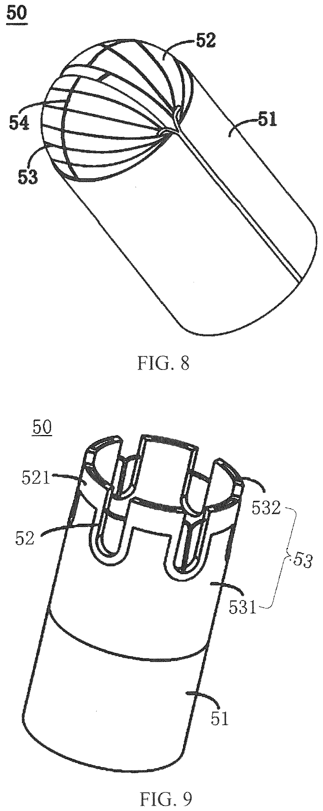

FIG. 9 is a further reference view showing the state of the sampling unit in the sampling system shown in FIG. 7.

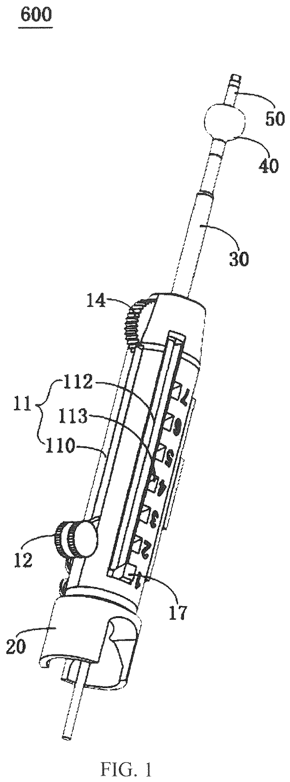

In FIGs. 1 to 10, 600 sampling system, 10 control unit, 20 endoscope connecting seat, 30 connecting tube, 40 balloon unit, 50 sampling unit, 11 housing, 12 first adjustment assembly, 13 linear rack, 14 second adjustment assembly, 15 balloon buffering assembly, 16 conductive component, 17 locking fin, 18 conducting assembly, 110 first opening, 111 second opening, 112 third opening, 113 clamping position, 114 blocking wall, 120 first adjustment knob, 121 connecting block, 140 second adjustment knob, 141 third adjustment gear, 150 magnetic damper, 151 mounting block, 152 spring, 160 cable sheath, 161 cable, 170 fixing portion, 171 clamping portion, 172 fixing hole, 180 body gas-liquid joint, 181 balloon gas-liquid joint, 182 gas-liquid tube connecting portion, 31 outer sheath, 32 gas-liquid tube, 33 communicating tube, 34 endoscope tube, 41 balloon, 42 metal ring, 51 sampling sheath, 52 support portion, 53 heating member, 54 support bar , 521 tongue, 531 first heating portion , 532 second heating portion , 55 outer ring , 56 inner ring, 57 heating piece, 58 metal strip, 59 insulating layer.

### DESCRIPTION OF THE EMBODIMENTS

For facilitating the understanding of the present application, the present application will be described in further detail below with reference to the drawings and specific embodiments. It should be noted that when an element is described as being "fixed to" another element, it may be directly provided on the said element, or there can be one or more intervening elements therebetween. When an element is described as being "connected" to another element, it may be directly connected to the said element, or there may be one or more intervening elements therebetween. The terms "vertical", "horizontal", "left", "right", "inner", "outer" and similar expressions used in this specification are for illustrative purposes only.

Unless defined otherwise, all technical and scientific terms used herein have the same meaning as commonly understood by a person skilled in the art to which the present application pertains. The terms used in the description of the present application are for the purpose of describing particular embodiments only and are not to limit the present application. The term "and/or" as used herein includes any and all combinations of one or more of the listed related items.

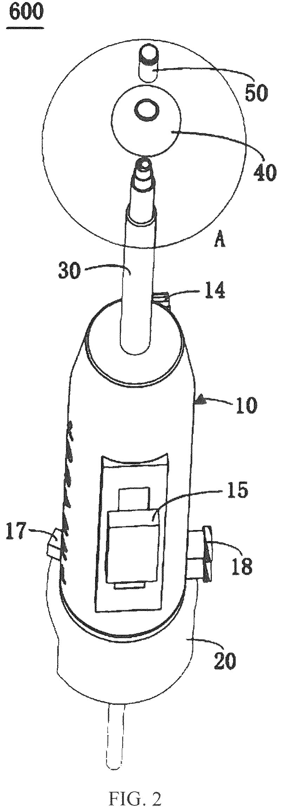

Referring to FIGs. 1 and 2, an embodiment of the present application provides a sampling system 600, which includes a control unit 10, an endoscope connecting seat 20, a connecting tube 30, a balloon unit 40 and a sampling unit 50. The control unit 10 is fixedly connected to the endoscope connecting seat 20. The connecting tube 30 has one end extending through the control unit 10 and is assembled thereto. The sampling unit 50 and the balloon unit 40 are both fixedly assembled to the connecting tube 30. The sampling unit 50 is located at one end of the connecting tube 30. The balloon unit 40 is located between the sampling unit 50 and the control unit 10. The sampling unit 50 and the balloon unit 40 is opposite to one end of the control unit 10, and the endoscope connecting seat 20 is located at the other end of control unit 10. In this embodiment, the control unit 10 is configured to adjust and control the working state and working position of the sampling unit 50 and the balloon unit 40.

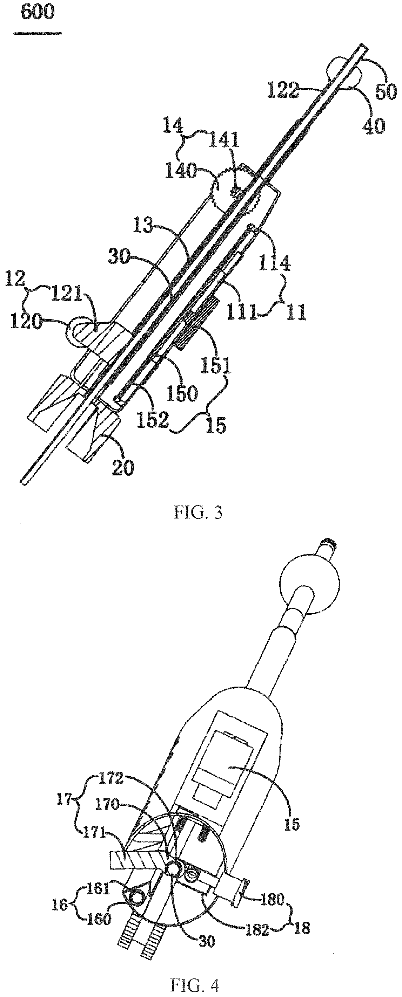

Referring to FIGs. 3 and 4, the control unit 10 includes a housing 11, a first adjustment assembly 12, a linear rack 13, a second adjustment assembly 14, a balloon buffering assembly 15, a conductive component 16, a locking fin 17, and a conducting assembly 18. The linear rack 13 is located inside the housing 11. The first adjustment assembly 12, the second adjustment assembly 14, the conductive component 16, the locking fin 17, the conducting assembly 18 and the balloon buffering assembly 15 are all partially located inside the housing 11 and partly located outside the housing 11.

The housing 11 is configured for installing and accommodating other parts of the control unit 10. The housing 11 can be made of a plastic material, which has a light weight and prevents electric leakage.

The housing 11 is substantially cylindrical with a chamfer at one end. Both ends of the housing 11 are each provided with an opening. The housing has a hollow structure inside for partially accommodating other parts of the control unit 10 and partially accommodating the connecting tube 30. The housing 11 is provided with a first opening 110, a second opening 111, a third opening 112, at least one clamping position 113 and at least one blocking wall 114. The first opening 110, the second opening 111, the third opening 112 and the clamping position 113 all penetrate the housing 11. The first opening 110, the second opening 111, and the third opening 112 are all rectangular holes; and the first opening 110, the second opening 111 and the third opening 112 all extend along the length direction of the housing 11.

The at least one clamping position 113 is located at one side of the third opening 112, and includes a plurality of clamping positions 113. The plurality of clamping positions 113 are arranged side by side along the longitudinal direction of the third opening 112, and communicated with the third opening 112.

The at least one blocking wall 114 is located inside the housing 11. In this embodiment, two blocking walls 114 are provided, and the two blocking walls 114 are located at two ends of the housing 11 respectively.

One end of the housing 11 is assembled with the endoscope connecting seat 20. Optionally, the housing 11 and the endoscope connecting seat 20 can be assembled by welding, or by threaded connection via a threaded hole.

In this embodiment, the endoscope connecting seat 20 is configured for mounting to other parts of the sampling system 600, so as to connect the housing 11 and other parts of the sampling system 600.

Further, the first adjustment assembly 12 is used to control the opening and closing of the sampling unit 50, and includes at least one first adjustment knob 120, at least one actuating string 122, and a connecting block 121, wherein two first adjustment knobs 120 are provided, and two actuating strings are provided correspondingly. The two first adjustment knobs 120 are both fitted to the connecting block 121, and are respectively located at two sides of the connecting block 121. The two first adjustment knobs 120 can rotate relative to the connecting block 121. The connecting block 121 is in contact with and connected to the connecting tube 30. The two actuating strings 122 are respectively fitted to the two first adjustment knobs 120. The controlling of the opening and closing of the sampling unit 50 can be achieved by rotating the two first adjustment knobs 120. For example, when the two first adjustment knobs 120 are rotated clockwise to wind the two actuating strings 122, the sampling unit 50 is opened. When the first adjustment knob 120 is rotated counterclockwise to loosen the two actuating strings 122, the sampling unit 50 is closed.

In this embodiment, the end of the actuating string 122 that is fitted to the sampling unit 50 is Y-shaped, and is fitted to the sampling unit 50 by welding or clamping.

The linear rack 13 is located inside the housing 11, and a portion of the connecting tube 30 is located inside the housing 11. The linear rack 13 is fixedly assembled with the portion of the connecting tube 30 located inside the housing 11. Further, the linear rack 13 is arranged along the length direction of and parallel to the connecting tube 30.

The second adjustment assembly 14 is configured to control the working positions of the sampling unit 50 and the balloon unit 40, and includes a second adjustment knob 140 and a third adjustment gear 141. The outer diameter of the second adjustment knob 140 is larger than that of the third adjustment gear 141, and the second adjustment knob 140 is assembled with the third adjustment gear 141. Optionally, the second adjustment knob 140 and the third adjustment gear 141 may be connected by a connecting rod. Alternatively, the second adjustment knob 140 and the third adjustment gear 141 may be assembled in other ways. The third adjustment gear 141 and the linear rack 13 are engaged with each other. When the second adjustment knob 140 is rotated, the third adjustment gear 141 is driven to rotate, thereby driving the linear rack 13 and the connecting tube 30 to move.

The balloon buffering assembly 15 is configured for assembly with an external structure during use, to fix the sampling system, or to stabilize the entire sampling system.

The balloon buffering assembly 15 includes a magnetic damper 150, a mounting block 151, and at least one spring 152. The magnetic damper 150 is assembled with the spring 152 and the mounting block 151, to compensate incidentally moving of the balloon unit 40 and the connecting tube 30, thus ensuring the balance of the entire sampling system 600.

Further, the magnetic damper 150 is in the form of a rectangular block structure, and the mounting block 151 is T-shaped. The magnetic damper 150 and the spring 152 are located inside the housing 11, and the mounting block 151 extends through the second opening 111 and is then fixedly assembled with the magnetic damper 150 located inside the housing 11. Optionally, the magnetic damper 150 and the mounting block 151 may be assembled by punching and screwing, or by other means.

In this embodiment, four springs 152 are provided, and the four springs 152 are all located in the housing 11 and are mounted in pairs on opposite two sides of the magnetic damper 150. Each spring 152 has one end abutting against the magnetic damper 150, and the other end abutting against the blocking wall 114.

In this embodiment, the housing 11 is further provided with a positioning member (not shown). The positioning member is located on the outer surface of the housing 11, and is fixed to the housing 11 and assembled with the balloon buffering assembly 15. The positioning member is also provided with an opening at a position aligned with the second opening 111. The mounting block 151 is partially extended through the opening in the positioning member and the second opening 111 and then fixedly assembled with the magnetic damper 150. The mounting block 151 is moveable relative to the positioning member.

Optionally, the positioning member has a reinforced connection and limiting function for the balloon buffering assembly 15. Of course, in other embodiments, the positioning member may be omitted.

Optionally, a plate-shaped wall of the housing 11 serves as a limiting wall (not shown), and the limiting wall together with another curved wall connected thereto defines a triangular receiving space. A cable 161 is partially received in the receiving space to prevent the cable 161 and other parts in the housing 11 from being in a mess.

Optionally, in some other embodiments, the conductive component 16 includes a cable sheath160 and the cable 161. The limiting wall can be omitted. The cable sheath 160 has a hollow structure with two open ends, and is installed in the housing 11. The cable 161 is partially coiled in the cable sheath 160, and the cable sheath 160 is used to prevent the cable 161 located in the housing 11 from being in a mess. One end of the cable 161 is electrically connected to an external circuit system, and the other end is connected to the sampling unit 50. The sampling unit 50 is electrically connected to a circuit and electrically conducted by the cable 16. During the sampling process, the tissues and blood vessels will inevitably be harmed, and the coagulation of blood can be accelerated by the conduction and heating of the sampling unit 50, to control the bleeding, and ensure the safety of the sampling process.

One end of the cable 161 is electrically connected to an external circuit, and the other end extends through the connecting tube 30 to reach the sampling unit 50. Optionally, a hole for running the cable 161 is provided in the connecting tube 30. The cable 161 runs through the hole in the connecting tube 30 to reach and be electrically connected to the sampling unit 50.

The locking fin 17 is fixedly assembled with the connecting tube 30, and used to clamped with the clamping position 113 of the housing 11.

Specifically, the locking fin 17 includes a fixing portion 170 and an engaging portion 171, with an obtuse angle formed therebetween. This arrangement avoids the interference of the locking fin 17 with the movement of the connecting tube 30, and facilitates the clamping between the locking fin 17 and the clamping position 113.

The fixing portion 170 is provided with a fixing hole 172. The fixing hole 172 extends from one side of the fixing portion 170 toward the clamping portion 171, and has a semicircular shape. The fixing hole 172 is configured for fixedly assembly with the connecting tube 30. Specifically, the fixing portion 170 is mounted around and tightly fitted with the connecting tube 30 via the fixing hole 172. In this manner, the fixing portion 170 is fixed to the connecting tube 30, and accordingly the locking fin 17 is fixed to the connecting tube 30, so that when the locking fin 17 moves, it can drive the connecting tube 30 to move.

In this embodiment, the fixing portion 170 is mounted around the connecting tube 30 via the fixing hole 172, and the fixing portion 170 restricts the position of the locking fin 17 with respect to the connecting tube 30 by at least one elastic retainer ring. Two elastic retainer rings are provided, which are respectively located at upper and lower ends of the fixing portion 170 to fix the locking fin 17 and the connecting tube 30.

The clamping portion 171 is configured to fit with the clamping position 113 to limit the position of the connecting tube 30 with respect to the housing 11, that is, to limit the working positions of the sampling unit 50 and the balloon unit 40 with respect to the housing 11. When the connecting tube 30 moves to a certain position, the clamping portion 171 is clamped at the clamping position 113, to restrict and fix the connecting tube 30. As a result, the connecting tube 30 will not move due to an external force, thereby restricting and fixing the working position of the balloon unit 40.

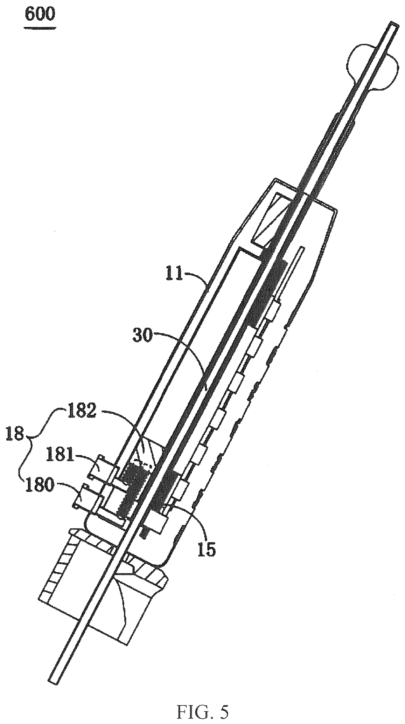

Referring to FIG. 5, the conducting assembly 18 includes a body gas-liquid joint 180, a balloon gas-liquid joint 181, and a gas-liquid tube connecting portion 182. The body gas-liquid joint 180 communicates with the internal environment of the human body, and the gas and liquid in the body is exported to an external environment by the body gas-liquid joint 180. The balloon gas-liquid joint 181 communicates with the balloon unit, and the gas and liquid in the balloon unit 40 is exported to an external environment by the balloon gas-liquid joint 181. The gas-liquid tube connecting portion 182 is used to store part of the gas and liquid from the body and the balloon unit.

The gas-liquid tube connecting portion 182 is fixedly connected to the connecting tube 30, and communicated with the connecting tube 30. Optionally, the gas-liquid tube connecting portion 182 and the connecting tube 30 are provided with holes, via which they communicated with each other.

The human body and the balloon unit 40 are communicated by the connecting tube 30. The gas-liquid tube connecting portion 182 is provided with a storage cavity (not shown). The storage cavity is optionally divided into two separate parts that are respectively in communication with the gas-liquid joint 180 and the balloon gas-liquid joint 181. A part of the gas-liquid tube connecting portion 182 receives the gas and liquid introduced from the body, and the other part receives the gas and liquid introduced from the balloon unit 40. The gas-liquid tube connecting portion 182 is further provided with at least one first gas-liquid vent (not shown) and at least one second gas-liquid vent (not shown).

In this embodiment, two first gas-liquid vents and two second gas-liquid vents are provided. The two first gas-liquid vents and the two second gas-liquid vents are located separately at two separate parts of the gas-liquid tube connecting portion 182. The connecting tube 30 and the gas-liquid tube connecting portion 182 are communicated by one of the first gas-liquid vents, and the gas-liquid tube connecting portion 182 and the body gas-liquid joint 180 are communicated by the other of the first gas-liquid vent, to enable gas and liquid conduction. The connecting tube 30 and the gas-liquid tube connecting portion 182 are communicated by one of the second gas-liquid vents, and the gas-liquid tube connecting portion 182 and the balloon gas-liquid joint 181 are communicated by the other of the second gas-liquid vents, to achieve gas and liquid conduction.

The body gas-liquid joint 180 is provided with a body gas-liquid exchange port (not shown) correspondingly, and the body gas-liquid exchange port communicates with the two first gas-liquid vents. By the body gas-liquid exchange port, the gas and liquid in the body are allowed to flow to the external environment, or the gas and liquid in the external environment is allowed to be introduced into the body.

The balloon gas-liquid joint 181 is provided with a balloon gas-liquid exchange port (not shown) correspondingly, and the balloon gas-liquid exchange port communicates with the two second gas-liquid vents. By the balloon gas-liquid exchange port, the gas and liquid in the balloon unit 40 are allowed to be introduced to the external environment, or the gas and liquid in the external environment is allowed to be introduced into the balloon unit 40.

Optionally, the balloon gas-liquid exchange port may be connected, by a tube, to an external device, for example, an oxygen supply device or a ventilation device.

Optionally, in this embodiment, a thin tube (not shown) is provided in the gas-liquid tube connecting portion 182 and the connecting tube 30. The thin tube has a hollow tubular structure with two open ends. The connecting tube 30 and the gas-liquid tube connecting portion 182 are provided with a hole for the thin tube extending through. The thin tube is partly located in the connecting tube 30, and partly located in the gas-liquid tube connecting portion 182. The thin tube allows the liquid and gas to flow between the connecting tube 30 and the gas-liquid tube connecting portion 182.

Referring to FIG. 6, the connecting tube 30 is made of a transparent material and includes an outer sheath 31, a gas-liquid tube 32, a communicating tube 33 and an endoscope tube 34. The outer sheath 31 is located at the outermost side, and the endoscope tube 34 is located at the innermost side. The gas-liquid tube 32 and the communicating tube 33 are located between the outer sheath 31 and the endoscope tube 34. Moreover, the gas-liquid tube 32 is closer to the outer sheath 31 than the communicating tube 33, and the communicating tube 33 is closer to the endoscope tube 34 than the gas-liquid tube 32. The length of the outer sheath 31 is shorter than those of the gas-liquid tube 32 and the communicating tube 33.

The outer sheath 31 protects other tubes inside, and one end of the outer sheath 31 is fixed to one end of the housing 11.

The gas-liquid tube 32 is used to conduct the liquid and gas. In this embodiment, the gas-liquid tube 32 communicates with the gas-liquid tube connecting portion 182 of the conducting assembly 18. Specifically, the gas-liquid tube 32 is provided with a first gas-liquid guide hole (not shown) and a second gas-liquid guide hole (not shown). The center line of the first gas-liquid guide hole is parallel to the center line of the second gas-liquid guide hole.

In this embodiment, the first gas-liquid guide hole communicates with the two first gas-liquid vents, and the first gas-liquid guide hole communicates with the balloon gas-liquid exchange port. The second gas-liquid guide hole communicates with the two second gas-liquid vents, and the second gas-liquid guide hole communicates with the body gas-liquid exchange port.

The communicating tube 33 is used to lead the actuating string 122 and the cable 161 to the sampling unit 50, so that the actuating string 122 can be assembled with the sampling unit 50, and the cable 161 and the sampling unit 50 can be electrically connected.

In this embodiment, the linear rack 13 is fixedly assembled with the communicating tube 33, and engaged with the third adjustment gear 141 of the second adjustment assembly 14. By rotating the second adjustment knob 140, the third adjustment gear 141 and the linear rack 13 interact with each other to drive the communicating tube 33 to move.

Optionally, in this embodiment, a plurality of receiving passages (not shown) and a plurality of apertures (not shown) are provided in the communicating tube 33. The plurality of receiving passages are used to receive the actuating string 122 and the cable 161, and the plurality of apertures are configured for the actuating string 122 and the cable 161 extending through, to realize the connection of the actuating string 122 and the cable 161 to the sampling unit 50.

The endoscope tube 34 is actually a part of an endoscope, and has one end equipped with a camera device for taking pictures of the environment in the body, so that the physician can judge the sampling state and the condition in the body.

In this embodiment, the endoscope tube 34 can move relative to the control unit 10. Specifically, the endoscope tube 34 can move relative to the housing 11 to take pictures of the environment in the body.

The balloon unit 40 includes a balloon 41 and a metal ring 42. The balloon 41 is made of a plastic material with an elastic deformation characteristic, and used to accommodate a gas and liquid. The metal ring 42 is used to prompt or indicate the position of the balloon 41. Optionally, the balloon 41 and the metal ring 42 are both mounted around the gas-liquid tube 32 at one end close to the sampling unit 50, without communicating with the balloon gas-liquid joint 181 and the body gas-liquid joint 180.

Specifically, in this embodiment, the metal ring 42 is mounted around the gas-liquid tube 32, and the gas-liquid tube 32 is provided with a first gas-liquid acquiring hole (not shown) at a position corresponding to the first gas-liquid guide hole.

In this embodiment, the gas and liquid in the balloon 41 flow in from the first gas-liquid acquiring hole of the gas-liquid tube 32, and flow to the balloon gas-liquid exchange port via the first gas-liquid guide hole and the two first gas-liquid vents in sequence and finally to the external environment or to the other device connected to the balloon gas-liquid joint 181. Alternatively, the gas and liquid can flow into the balloon unit 40 from a reverse direction.

When sampling, the gas and liquid in the body flow in from the second gas-liquid guide hole, and flow to body gas-liquid exchange port via the two second gas-liquid vents and finally to the external environment. Alternatively, the gas and liquid can flow into the body from a reverse direction.

In this embodiment, the fixing portion 170 is mounted around the gas-liquid tube 32 through the fixing hole 172, and closely fits with the gas-liquid tube 32, to fix the fixing portion 170 and the gas-liquid tube 32. That is, the locking fin 17 and the gas-liquid tube 32 are fixed, so that the gas-liquid tube 32 can temporarily lock the positions of the balloon 41 and the gas-liquid tube 32 before the balloon 41 expands. After the balloon 41 expands, the locking of the position of the locking fin 17 can be released.

Referring to FIGs. 7 and 8, the sampling unit 50 includes a sampling sheath 51, a support portion 52, a heating member 53 and at least one support bar 54.

The sampling sheath 51 is mounted around the endoscope tube 34 and assembled with the endoscope tube 34. Moreover, the sampling sheath 51 is made of a relatively hard material, and used to install the support portion 52, to support and protect the support portion 52.

In this embodiment, the support portion 52 is a plastic molded member that is deformable by an external force, and is made of a plastic material with a certain hardness. The support portion 52 includes two parts, which are located on two sides of the same end of the sampling sheath 51. Two support bars 54 are provided, and the two support bars 54 are respectively assembled with the two parts of the support portion 52, to support the support portion 52, so that the support portion 52 can maintain its shape after being expanded.

Optionally, there may be more than two support bars 54.

The heating member 53 is mounted around the support portion 52. Optionally, a part of the heating member 53 is inserted and fixed in the sampling sheath 51, and the other part is mounted around the support portion 52.

It should be noted that in this embodiment, the heating member 53 also includes two parts. The two parts of the heating member 53 are respectively mounted around the outer surfaces of the two parts of the support portion 52. The two parts of the heating member 53 are fixedly assembled with the actuating strings 122, and the entire heating member 53 is opened and closed by controlling the movement of the actuating strings 122.

The heating member 53 is a heating coil, and the heating coil includes two parts. The two parts of the heating coil are respectively mounted around the two parts of the support portion 52. The two parts of the heating coil are deformable upon an external force. Normally, the two parts of the heating coil are wound into a cylindrical shape. One end of the actuating string 122 is assembled with the ends of the two parts of the heating coil that is away from the sampling sheath 51. Optionally, one end of the actuating string 122 is assembled with the ends of the heating coil away from the sampling sheath 51 by hooking.

When the first adjustment knob 120 is rotated clockwise, the actuating string 122 is pulled. The two parts of the heating coil are pulled, such that the entire heating member 53 is in a hemispheric shape (as shown in FIG. 8), the heating member 53 is closed, and the heating member 53 drives the support portion 52 to close at the same time. When the first adjustment knob 120 is rotated counterclockwise, the actuating string 122 is loosened, and without a pulling force, the two parts of the heating coil is unstressed, and returns to a cylindrical shape (as shown in FIG. 7). As a result, the heating member 53 opens, and the heating member 53 drives the support portion 52 to open.

When the heating member 53 is opened and closed, it drives the support portion 52 to open and close. The heating member 53 is connected to the cable 161 to realize electrical connection with an external circuit and realize heat generation. In this manner, the bleeding can be controlled, ensuring the safety of the sampling process.

It should be noted that in this embodiment, it is the heating member 53, instead of the support portion 52, that is used to cut the tissue. The support portion 52 is used to support the heating member 53, to prevent the heating member 53 from being in a mess, and serves to collect the tissues cut by the heating member 53. Referring to FIG. 9, in some other embodiments, the support portion 52 is a plastic molded member. One end of the support portion 52 is fixedly assembled with the sampling sheath 51. The support portion 52 is electrically insulating and has a cylindrical shape. The end of the support portion 52 away from the sampling sheath 51 is cut to form a plurality of tongues 521, and the tongues 521 are electrically insulating. The heating member 53 includes a first heating portion 531 and a second heating portion 532. The first heating portion 531 is similar in shape to the support portion 52, and is mounted around the outer surface of the support portion 52. The second heating portion 532 is embedded in the tongue 521. The actuating string 122 is in the form of a lasso. The actuating string 122 is partially embedded in the support portion 52, and the portion of the actuating string 122 embedded in the support portion 52 is annular. A portion of the actuating string 122 runs along the connecting tube 30 through the support portion 52, and extends to the first adjustment assembly 12 for assembly with the first adjustment knob 120.

Optionally, the support bar 54 may not be rigid.

When the first adjustment knob 120 is rotated clockwise, the actuating string 122 is pulled, and the tongues 521 on the support portion 52 are pulled at the same time. The tongues 521 are bent in the same direction, so that the entire support portion 52 is in a closed state. When the first adjustment knob 120 is rotated counterclockwise, the actuating string 122 is unstressed, so that the tongues 521 are subjected to no force, and thus returns to its initial shape, and slowly becomes straight, making the entire support portion 52 in an open state.

Optionally, a non-thermal conductive layer (not shown) is provided between the first heating portion 531 and the sampling sheath 51 to separate the sampling sheath 51 and the first heating portion 531 to protect the sampling sheath 51 against influence by the first heating portion 531.

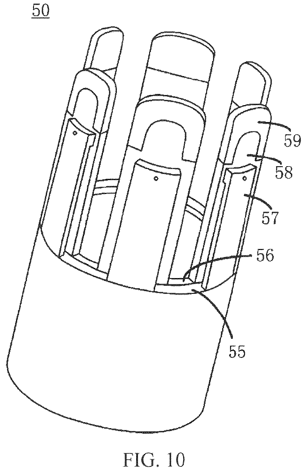

Referring to FIG. 10, in another embodiment, the sampling unit 50 does not include the sampling sheath 51, the support portion 52, the heating member 53 and the support bar 54. Instead, the sampling unit 50 includes an outer ring 55, an inner ring 56, at least one heating piece 57, at least one metal strip 58, and an insulating layer 59.

The outer ring 55 and the inner ring 56 are both connected to the communicating tube 33, and the outer ring 55 and the inner ring 56 are both rotatable relative to the communicating tube 33.

Optionally, the communicating tube 33, the outer ring 55 and the inner ring 56 are provided with a receiving passage (not shown) for receiving the actuating string 122. The outer ring 55 and the inner ring 56 each have a hollow cylindrical shape with two open ends. The outer ring 55 and the inner ring 56 each are further provided with an annular tube (not shown) respectively at the end away from the communicating tube 33. The receiving passages are perpendicular to the annular tubes. One end of the actuating string runs through the communicating tube 33, and the receiving passages of the outer ring 55 and the inner ring 56, and is then fixed in the annular tubes. Optionally, the annular tubes of the outer ring 55 and the inner ring 56 each are provided with an opening (not shown).

In this embodiment, two actuating strings 122 are provided, which are respectively an outer ring actuating string and an inner ring actuating string. The sampling system 600 is provided with a gear (not shown) for controlling the actuating string. Two gears are provided, which are both installed on the control unit 10, and are respectively an outer ring gear and an inner ring gear.

The outer ring gear is used to control the state of the outer ring actuating string. The outer ring actuating string surrounds the outer ring gear, and two ends of the outer ring actuating string respectively pass through the receiving passage and enter the annular tube from the opening of the annular tube of the outer ring. The two ends enter the annular tube in two opposite directions, and are both fixed in the annular tube so that the portion of the outer ring actuating string located in the annular tube is ring-shaped.

By rotating the outer ring gear, the positions of two portions of the actuating string respectively on two sides of the outer ring gear is controlled, to control the positions of the two ends of the outer ring actuating string, so as to rotate the outer ring 55.

Similarly, the inner ring gear is used to control the state of the inner ring actuating string. The inner ring actuating string surrounds the inner ring gear, and two ends of the inner ring actuating string respectively pass through the receiving passage and enter the annular tube from the opening of the annular tube of the inner ring. The two ends enter the annular tube in two opposite directions, and are both fixed in the annular tube so that the portion of the inner ring actuating string located in the annular tube is ring-shaped.

By rotating the inner ring gear, the positions of two portions of the actuating string respectively on two sides of the inner ring gear is controlled, to control the positions of the two ends of the inner ring actuating string, so as to rotate the outer ring 56.

Further, by rotating the outer ring gear, the inner ring gear can be driven to rotate in an opposite direction. Therefore, in this embodiment, the inner ring gear and the outer ring gear rotate in opposite directions.

Further, one end of the heating piece 57 is fixed to one end of the outer ring 55, and one end of the metal strip 58 is fixed to one end of the inner ring 56. Optionally, six heating pieces 57 and six metal strips 58 are provided, and the six heating pieces 57 and six metal strips 58 are annularly arranged. Of course, other number of heating pieces 57 and metal strips 58 can be provided, which are not limited herein.

The sampling unit 50 also includes a fastener (not shown). Optionally, six fasteners are provided. Each heating piece 57 and one corresponding metal strip 58 are fixedly connected by one fastener. In addition, the heating piece 57 and the metal strip 58 are rotatable, and the fastener is located on an end of the heating piece 57 away from the outer ring 55.

The insulating layer 59 is mounted on the metal strip 58 to provide insulation and protection. Optionally, an air insulating layer is provided between the heating piece 57 and the metal strip 58.

The outer ring gear and the inner ring gear are rotated, and the outer ring 55 and the inner ring 56 are driven to move by controlling the rotation positions of the two ends of the outer ring actuating string and the inner ring actuating string. When the outer ring 55 and the inner ring 56 rotate in opposite directions, the heating piece 57 and the metal strip 58 are driven to rotate in opposite directions. The heating piece 57 and the metal strip 58 are fixed by the fastener, so that the heating piece 57 and the metal strip 58 are bent. The heating piece 57 and the metal strip 58 are both bent in the same direction, so that the sampling unit 50 is closed, and sampling is implemented. Correspondingly, when the outer ring gear and the inner ring gear are rotated, the outer ring 55 and the inner ring 56 rotate in the reverse directions, the heating piece 57 and the metal strip 58 rotate in the other directions, and then the heating piece 57 and the metal strip 58 slowly return to their original shape, so that the sampling unit 50 is opened. In this embodiment, an assembling procedure is to assemble the balloon unit 40 and the connecting tube 30, then assemble the sampling unit 50 and the connecting tube 30, next assemble the connecting tube 30 and the control unit 10, and finally assemble the endoscope connecting seat 20 and the control unit 10.

In the technical solution of the present application, the sampling system 600 includes the control unit 10, the connecting tube 30, and the sampling unit 50. The control unit 10 and the sampling unit 50 are both assembled with the connecting tube 30; and the control unit 10 is provided with the first adjustment assembly 12, and the first adjustment assembly 12 is provided with the first adjustment knob 120, the connecting block 121, and the actuating string. The actuating string is rigid, the first adjustment knob 120 and the connecting block 121 are assembled, and the first adjustment knob 120 is rotatable. The sampling unit 50 has the sampling sheath 51, the support portion 52, and the heating member 53. The sampling sheath 51 and the connecting tube 30 are fixedly assembled, the support portion 52 and the sampling sheath 51 are assembled, and the heating member 53 is mounted around the support portion 52. One end of the actuating string is assembled with the first adjustment knob 120, and the other end is fixed with the heating member 53. By rotating the first adjustment knob 120, the actuating string is wound or loosened, to control the heating member 53 to be opened or closed, thereby realizing the cutting and acquisition of internal tissues, with convenient, safe and reliable operations.

Further, the control unit 10 further includes the conductive component 16, and the conductive component 16 includes the cable 161. The cable 161 is connected to the heating member 53 through the connecting tube 30, so the heating member 53 can be energized to generate heat, thereby controlling the bleeding and ensuring the safety of the sampling process.

Further, the sampling system 600 further includes the balloon unit 40, and the control unit 10 is also provided with the second adjustment assembly 14 and the linear rack 13. The second adjustment assembly 14 is engaged with the linear rack 13. By adjusting the second adjustment assembly 14, the position of the sampling unit 50 relative to the control unit 10 can be controlled, to remain the positions of the gas-liquid tube 32 and the balloon unit 40.

It should be noted that preferred embodiments of the present application are given in the specification and drawings of the present application. However, the present application can be implemented in many different forms and, is not limited to the embodiments described in the specification. These embodiments cause no additional restrictions on the disclosure of the present application, are provided for thorough and comprehensive understanding of the disclosure of the present application. In addition, the above technical features can be combined with each other to form various embodiments not listed above, which are all contemplated in the scope of the description of the present disclosure. Further, modifications and changes can be made by those skilled in the art in accordance with the above description. The scope of the invention is defined by the appended claims solely.

## Claims

1. A sampling system (600), comprising:
a sampling unit (50), configured for acquiring a tissue sample;
a control unit (10), configured for controlling a working state of the sampling unit (50) and comprising a first adjustment assembly (12), wherein the first adjustment assembly (12) comprises a first adjustment knob (120) and an actuating string (122), and the actuating string (122) is rigid; and
a connecting tube (30), assembled to the control unit (10) and having at least one end running through the control unit (10), and one end of the connecting tube (30) being fixedly assembled to the sampling unit (50),
wherein one end of the actuating string (122) is fixed to the first adjustment knob (120) and an other end of the actuating string (122) is fixed to the sampling unit (50); and due to the rigidity of the actuating string (122) the sampling unit (50) is controllable to be opened or closed by controlling the first adjustment knob (120), wherein
the sampling unit (50) comprises a sampling sheath (51), a support portion (52) and a heating member (53), wherein one end of the sampling sheath (51) inserts into the connecting tube (30) and connected to the connecting tube (30), and an other end of the sampling sheath (51) is connected to the support portion (52); the support portion (52) is deformable upon an external force, and the heating member (53) is mounted around the sampling sheath (51),
the one end of the actuating string (122) is fixed to the first adjustment knob (120) and the other end of the actuating string (122) is fixed to the heating member (53); the actuating string (122) is controllable to move by controlling the first adjustment knob (120), whereby the heating member (53) is controlled to be opened or closed, which in turn drives the support portion (52) to be opened or closed,
the connecting tube (30) comprises a communicating tube (33), having at least one end extending through the control unit (10), wherein the one end of the sampling sheath (51) inserts into the communicating tube (33) and is connected to the communicating tube (33),
the communicating tube (33) is provided with a receiving passage and an aperture, wherein the aperture is located in the control unit (10),
the connecting tube (30) further comprises an endoscope tube (34) and an outer sleeve,
wherein
at least one end of the endoscope tube (34) extends through the control unit (10), and the outer sleeve is located outside the control unit and has one end assembled to the control unit, the outer sleeve is mounted around the communicating tube, and the communicating tube is mounted around the endoscope tube; **characterized in that**
one end of the endoscope tube (34) is assembled to the sampling sheath (51), the endoscope tube (34) is movable relative to the control unit (10), and
the sampling system (600) further comprises a balloon unit (40) having a balloon (41) and a metal ring (42), wherein the balloon (41) is mounted around the metal ring (42), the metal ring (42) is mounted around the connecting tube (30), and the balloon unit (40) is located between the sampling unit (50) and the control unit (10).

2. The sampling system (600) according to claim 1, wherein
the control unit (10) comprises a conductive component (16) having a cable sheath (160) and a cable (161), wherein
the cable sheath (160) is fixedly mounted in the control unit (10), and the cable (161) is partly located in the cable sheath (160) and partly in the receiving passage, and the cable (161) has one end electrically connected to the outside and an other end extending through the aperture and the receiving passage and electrically connected to the heating member (53).

3. The sampling system (600) according to claim 1, wherein
the connecting tube (30) further comprises a gas-liquid tube (32) located between the outer sleeve and the communicating tube (33), and
both the balloon (41) and the metal ring (42) are mounted around one end of the gas-liquid tube (32) adjacent to the sampling unit (50).

4. The sampling system (600) according to claim 3, wherein
the control unit (10) further comprises a linear rack (13) and a second adjustment assembly (14),
the linear rack (13) is fixed to the communicating tube (33) and the linear rack (13) is parallel to the communicating tube (33); and
the second adjustment assembly (14) comprises a second adjustment knob (140) and a third adjustment gear (141), the second adjustment knob (140) is assembled to the third adjustment gear (141), and the third adjustment gear (141) is drivable by the second adjustment knob (140) to rotate; and
wherein the second adjustment knob (140) is assembled to the control unit (10), and the second adjustment knob (140) is partially located in the control unit (10); and the third adjustment gear (141) is located in the control unit (10) and engaged with the linear rack (13).

5. The sampling system (600) according to claim 3, wherein
the control unit (10) further comprises a conducting assembly (18) having a balloon gas-liquid joint (181), wherein the balloon gas-liquid joint (181) is communicated with the gas-liquid tube (32), and the balloon gas-liquid joint (181) is provided with a balloon gas-liquid exchange port thereon;
the gas-liquid tube (32) is provided with a first gas-liquid guide hole;
an inner wall of the balloon (41) is connected to the gas-liquid tube (32) and is provided with a first gas-liquid acquiring hole; and
the first gas-liquid acquiring hole communicates with the first gas-liquid guide hole, and the balloon gas-liquid exchange port communicates with the first gas-liquid guide hole and an external environment.

6. The sampling system (600) according to claim 5, wherein
the conducting assembly (18) further comprises a body gas-liquid joint (180), wherein the body gas-liquid joint (180) is connected to the gas-liquid tube (32), and the body gas-liquid joint (180) is provided with a body gas-liquid exchange port thereon;
the gas-liquid tube (32) is provided with a second gas-liquid guide hole, and the second gas-liquid guide hole communicates with an internal environment; and
the second gas-liquid guide hole communicates with the body gas-liquid exchange port, and the body gas-liquid exchange port communicates with the second gas-liquid guide hole and the external environment.

## Patentansprüche

1. Ein Probennahmesystem (600), umfassend:
eine Probennahmeeinheit (50), die konfiguriert ist, um eine Gewebeprobe zu erhalten;
eine Steuereinheit (10), die konfiguriert ist, um einen Arbeitszustand der Probennahmeeinheit (50) zu steuern und die eine erste Verstellbaugruppe umfasst, wobei die erste Verstellbaugruppe (12) einen ersten Verstellregler (120) und eine Auslöseschnur (122) umfasst, und die Auslöseschnur (122) ist starr; und
ein Verbindungsrohr (30), das an der Steuereinheit (10) montiert ist, wobei sich mindestens ein Ende davon durch die Steuereinheit (10) erstreckt, und ein Ende des Verbindungsrohrs (30) ist fest an der Probennahmeeinheit (50) montiert,
wobei ein Ende der Auslöseschnur (122) an dem ersten Verstellregler (120) befestigt ist und ein andere Ende der Auslöseschnur (122) ist an der Probennahmeeinheit (50) befestigt; und aufgrund der Steifigkeit der Auslöseschnur (122) ist die Probennahmeeinheit (50) so steuerbar, dass sie durch Steuern des ersten Verstellreglers (120) geöffnet und geschlossen werden kann, wobei die Probennahmeeinheit (50) eine Probennahmeummantelung (51), einen Tragebereich (52) und ein Heizelement (53) umfasst, wobei ein Ende der Probennahmeummantelung (51) in das Verbindungsrohr eingesetzt ist und mit dem Verbindungsrohr (30) verbunden ist, und ein anderes Ende der Probennahmeummantelung (51) ist mit dem Tragebereich (52) verbunden; der Tragebereich (52) ist durch eine äußere Kraft verformbar, und das Heizelement (53) ist um die Probennahmeummantelung (51) angeordnet,
das eine Ende der Auslöseschnur (122) ist an dem ersten Verstellregler (120) befestigt und das andere Ende der Auslöseschnur (122) ist an dem Heizelement (53) befestigt; die Auslöseschnur (122) ist durch Steuern des ersten Verstellreglers (120) steuerbar, sich zu bewegen, wobei das Heizelement (51) so gesteuert wird, dass es geöffnet oder geschlossen ist, wodurch wiederum der Tragebereich (52) veranlasst wird, geöffnet oder geschlossen zu sein,
das Verbindungsrohr (30) umfasst ein Übermittlungsrohr (33), das mindestens ein Ende aufweist, das sich durch die Steuereinheit (10) erstreckt, wobei das eine Ende der Probennahmeummantelung (51) in das Übermittlungsrohr (33) eingreift und mit dem Übermittlungsrohr (33) verbunden ist, das Übermittlungsrohr (33) ist mit einem Aufnahmedurchlass und einer Öffnung versehen, wobei sich die Öffnung in der Steuereinheit (10) befindet,
das Verbindungsrohr (30) umfasst des Weiteren ein Endoskoprohr (34) und eine Außenhülse, wobei sich mindestens ein Ende des Endoskoprohrs (34) durch die Steuereinheit (10) erstreckt und sich die Außenhülse außerhalb der Steuereinheit befindet und ein Ende aufweist, das an der Steuereinheit montiert ist, die Außenhülse ist um das Übermittlungsrohr herum montiert und das Übermittlungsrohr ist um das Endoskoprohr herum montiert, **dadurch gekennzeichnet, dass**
ein Ende des Endoskoprohrs (34) an der Probennahmeummantelung (51) angeordnet ist, das Endoskoprohr (34) bezogen auf die Steuereinheit (10) beweglich ist und das Probennahmesystem (600) des Weiteren eine Balloneinheit (40) umfasst, die einen Ballon (41) und einen Metallring (42) aufweist, wobei der Ballon (41) um den Metallring (42) herum angeordnet ist, der Metallring (42) um das Verbindungsrohr (30) herum angeordnet ist, und sich die Balloneinheit (40) zwischen der Probennahmeeinheit (50) und der Steuereinheit (10) befindet.

2. Das Probennahmesystem (600) gemäß Anspruch 1, wobei
die Steuereinheit (10) ein leitendes Bauteil (16) umfasst, das eine Kabelummantelung (160) und ein Kabel (161) aufweist, wobei
die Kabelummantelung (160) fest in der Steuereinheit (10) montiert ist, und das Kabel (161) befindet sich zum Teil in der Kabelummantelung (160) und zum Teil in dem Aufnahmedurchlass, und das Kabel (161) weist ein Ende auf, das elektrisch mit der Außenseite verbunden ist und ein anderes Ende, das sich durch die Öffnung und den Aufnahmedurchlass erstreckt und elektrisch mit dem Heizelement (53) verbunden ist.

3. Das Probennahmesystem (600) gemäß Anspruch 1, wobei
das Verbindungsrohr (30) weiterhin ein Gas-Flüssigkeitsrohr (32) umfasst, das sich zwischen der Außenhülse und dem Übermittlungsrohr (33) befindet, und
sowohl der Ballon (41) und der Metallring (42) um ein Ende des Gas-Flüssigkeitsrohrs (32) angrenzend an die Probennahmeeinheit (50) angeordnet sind.

4. Das Probennahmesystem (600) gemäß Anspruch 3, wobei
die Steuereinheit (10) des Weiteren einen linearen Träger (13) und eine zweite Verstellbaugruppe (14) umfasst,
der lineare Träger (13) an dem Übermittlungsrohr (33) befestigt ist und der lineare Träger parallel zu dem Übermittlungsrohr (33) angeordnet ist; und
die zweite Verstellbaugruppe (14) umfasst einen zweiten Verstellregler (140) und ein drittes Verstellzahnrad (141), der zweite Verstellregler (140) ist an dem dritten Verstellzahnrad (141) angeordnet, und das dritte Verstellzahnrad (141) ist von dem zweiten Verstellregler (140) zur Drehung antreibbar; und
wobei der zweite Verstellregler (140) an der Steuereinheit (10) angeordnet ist, und der zweite Verstellregler (140) befindet sich zum Teil in der Steuereinheit (10); und das dritte Verstellzahnrad (141) befindet sich in der Steuereinheit (10) und wird von dem linearen Träger (13) in Eingriff genommen.

5. Das Probennahmesystem (600) gemäß Anspruch 3, wobei
die Steuereinheit (10) des Weiteren eine leitende Baugruppe (18) umfasst, die eine Ballon-Gas-Flüssigkeitsverbindung (181) aufweist, wobei die Ballon-Gas-Flüssigkeitsverbindung (181) mit dem Gas-Flüssigkeitsrohr (32) in Verbindung steht, und die Ballon-Gas-Flüssigkeitsverbindung (181) ist mit einem daran angeordneten Ballon-Gas-Flüssigkeitsaustauschanschluss versehen;
die Gas-Flüssigkeitsleitung (32) ist mit einem daran angeordneten ersten Gas-Flüssigkeitsleitungsloch versehen;
eine Innenwand des Ballons (41) ist mit dem Gas-Flüssigkeitsrohr (32) verbunden und mit einem ersten Gas- Flüssigkeitsbeschaffungsloch versehen; und
das erste Gas-Flüssigkeitsbeschaffungsloch steht mit dem ersten Gas-Flüssigkeitsführungsloch in Verbindung, und der Ballon-Gas-Flüssigkeitsaustauschanschluss steht mit dem ersten Gas-Flüssigkeitsführungsloch und einer Außenumgebung in Verbindung.

6. Das Probennahmesystem (600) gemäß Anspruch 5, wobei
die leitende Baugruppe (18) des Weiteren eine Körper-Gas-Flüssigkeitsverbindung (180) aufweist, wobei die Körper-Gas-Flüssigkeitsverbindung (180) mit dem Gas-Flüssigkeitsrohr (32) verbunden ist, und die Körper-Gas-Flüssigkeitsverbindung (180) ist mit einem daran angeordneten Körper-Gas-Flüssigkeitsaustauschanschluss versehen;
die Gas-Flüssigkeitsleitung (32) ist mit einem zweiten Gas-Flüssigkeitsführungsloch versehen, und das zweite Gas-Flüssigkeitsführungsloch steht mit einer Innenumgebung in Verbindung; und das zweite Gas-Flüssigkeitsführungsloch steht mit dem Körper-Gas-Flüssigkeitsaustauschanschluss in Verbindung, und der Körper-Gas-Flüssigkeitsaustauschanschluss steht mit dem zweiten Gas-Flüssigkeitsführungsloch und der Außenumgebung in Verbindung.

## Revendications

1. Système d'échantillonnage (600) comportant :
une unité d'échantillonnage (50), conçue pour acquérir un échantillon de tissu ;
une unité de commande (10), configurée pour commander un état de fonctionnement de l'unité d'échantillonnage (50) et comportant un premier ensemble de réglage (12), dans lequel le premier ensemble de réglage (12) comporte une première molette de réglage (120) et une chaîne d'actionnement (122) et la chaîne d'actionnement (122) est rigide ; et
un tube de liaison (30), assemblé avec l'unité de commande (10) et ayant au moins une extrémité passant à travers l'unité de commande (10) et une extrémité du tube de liaison (30) étant assemblée à demeure avec l'unité d'échantillonnage (50),
dans lequel une extrémité de la chaîne d'actionnement (122) est fixée à la première molette de réglage (120) et une autre extrémité de la chaîne d'actionnement (122) est fixée à l'unité d'échantillonnage (50) ; et en raison de la rigidité de la chaîne d'actionnement (122), l'unité d'échantillonnage (50) peut être commandée pour être ouverte ou fermée par commande de la première molette de réglage (120), dans lequel
l'unité d'échantillonnage (50) comporte une gaine d'échantillonnage (51), une portion de support (52) et un élément de chauffage (53), dans lequel une extrémité de la gaine d'échantillonnage (51) s'insère dans le tube de liaison (30) et est reliée au tube de liaison (30) et une autre extrémité de la gaine d'échantillonnage (51) est reliée à la portion de support (52) ; la portion de support (52) est déformable sous l'effet d'une force externe et l'élément de chauffage (53) est monté autour de la gaine d'échantillonnage (51),
la première extrémité de la chaîne d'actionnement (122) est fixée à la première molette de réglage (120) et l'autre extrémité de la chaîne d'actionnement (122) est fixée à l'élément de chauffage (53) ; la chaîne d'actionnement (122) peut être commandée pour se déplacer par commande de la première molette de réglage (120), moyennant quoi l'élément de chauffage (53) est commandé pour être ouvert ou fermé, ce qui entraîne ensuite la portion de support (52) afin qu'elle soit ouverte ou fermée,
le tube de liaison (30) comporte un tube de communication (33), ayant au moins une extrémité s'étendant à travers l'unité de commande (10), dans lequel la première extrémité de la gaine d'échantillonnage (51) s'insère dans le tube de communication (33) et est reliée au tube de communication (33), le tube de communication (33) est pourvu d'un passage de réception et d'une ouverture, dans lequel l'ouverture est située dans l'unité de commande (10),
le tube de liaison (30) comporte en outre un tube d'endoscope (34) et un manchon extérieur, dans lequel
au moins une extrémité du tube d'endoscope (34) s'étend à travers l'unité de commande (10), et le manchon extérieur est situé à l'extérieur de l'unité de commande et a une extrémité assemblée avec l'unité de commande, le manchon extérieur est monté autour du tube de communication, et le tube de communication est monté autour du tube d'endoscope ; **caractérisé en ce que**
une extrémité du tube d'endoscope (34) est assemblée avec la gaine d'échantillonnage (51), le tube d'endoscope (34) est mobile par rapport à l'unité de commande (10), et
le système d'échantillonnage (600) comporte en outre une unité ballonnet (40) ayant un ballonnet (41) et une bague métallique (42), dans lequel le ballonnet (41) est monté autour de la bague métallique (42), la bague métallique (42) est montée autour du tube de liaison (30) et l'unité ballonnet (40) est située entre l'unité d'échantillonnage (50) et l'unité de commande (10).

2. Système d'échantillonnage (600) selon la revendication 1, dans lequel
l'unité de commande (10) comporte un composant conducteur (16) ayant une gaine de câble (160) et un câble (161), dans lequel
la gaine de câble (160) est montée à demeure dans l'unité de commande (10) et le câble (161) est situé partiellement dans la gaine de câble (160) et partiellement dans le passage de réception, et le câble (161) a une extrémité raccordée électriquement à l'extérieur et une autre extrémité s'étendant à travers l'ouverture et le passage de réception et raccordée électriquement à l'élément de chauffage (53).

3. Système d'échantillonnage (600) selon la revendication 1, dans lequel
le tube de liaison (30) comporte en outre un tube de gaz-liquide (32) situé entre le manchon extérieur et le tube de communication (33), et
à la fois le ballonnet (41) et la bague métallique (42) sont montés autour d'une extrémité du tube de gaz-liquide (32) adjacente à l'unité d'échantillonnage (50).

4. Système d'échantillonnage (600) selon la revendication 3, dans lequel
l'unité de commande (10) comporte en outre une baie linéaire (13) et un second ensemble de réglage (14),
la baie linéaire (13) est fixée au tube de communication (33) et la baie linéaire (13) est parallèle au tube de communication (33) ; et
le second ensemble de réglage (14) comporte une seconde molette de réglage (140) et un troisième engrenage de réglage (141), la seconde molette de réglage (140) est assemblée avec le troisième engrenage de réglage (141) et le troisième engrenage de réglage (141) peut être entraîné par la seconde molette de réglage (140) pour entrer en rotation ; et
dans lequel la seconde molette de réglage (140) est assemblée à l'unité de commande (10) et la seconde molette de réglage (140) est située partiellement dans l'unité de commande (10) ; et le troisième engrenage de réglage (141) est situé dans l'unité de commande (10) et mis en prise avec la baie linéaire (13).

5. Système d'échantillonnage (600) selon la revendication 3, dans lequel
l'unité de commande (10) comporte en outre un ensemble conducteur (18) ayant un joint de gaz-liquide de ballonnet (181), dans lequel le joint de gaz-liquide de ballonnet (181) est mis en communication avec le tube de gaz-liquide (32) et le joint de gaz-liquide de ballonnet (181) est pourvu d'un orifice d'échange de gaz-liquide de ballonnet sur lui ;
le tube de gaz-liquide (32) est pourvu d'un premier trou de guidage de gaz-liquide ;
une paroi interne du ballonnet (41) est reliée au tube de gaz-liquide (32) et est pourvue d'un premier trou d'acquisition de gaz-liquide ; et
le premier trou d'acquisition de gaz-liquide communique avec le premier trou de guidage de gaz-liquide et l'orifice d'échange de gaz-liquide de ballonnet communique avec le premier trou de guidage de gaz-liquide et un environnement externe.

6. Système d'échantillonnage (600) selon la revendication 5, dans lequel
l'ensemble conducteur (18) comporte en outre un joint de gaz-liquide de corps (180), dans lequel le joint de gaz-liquide de corps (180) est relié au tube de gaz-liquide (32) et le joint de gaz-liquide de corps (180) est pourvu d'un orifice d'échange de gaz-liquide de corps sur lui ;
le tube de gaz-liquide (32) est pourvu d'un second trou de guidage de gaz-liquide et le second trou de guidage de gaz-liquide communique avec un environnement interne ; et
le second trou de guidage de gaz-liquide communique avec l'orifice d'échange de gaz-liquide de corps et l'orifice d'échange de gaz-liquide de corps communique avec le second trou de guidage de gaz-liquide et l'environnement externe.
